# EUROPÄISCHE PATENTANMELDUNG

(11) **EP 2 123 454 A1**
(43) Veröffentlichungstag der Anmeldung: **25.11.2009**
(21) Anmeldenummer: 09160220.1
(22) Anmeldetag: 14.05.2009
(51) Int. Cl.: B41F 13/00, B41F 13/008, B41F 33/00, F16N 29/00, G01N 33/28

(54) **Verfahren zum Betreiben einer Druckmaschine**

(30) Priorität: 21.05.2008 DE 102008024616
(71) Anmelder: Manroland AG, 63075 Offenbach am Main (DE)
(72) Erfinder: Albrecht, Stefan, 86356, Neusäß (DE); Schall, Nils-Hendric, 86153, Augsburg (DE); Weng, Markus, 86733, Alerheim (DE)

(57) **Zusammenfassung**

Die Erfindung betrifft ein Verfahren zum Betreiben einer Druckmaschine, wobei im Betrieb der Druckmaschine geschmierte Baugruppen derselben derart mit Schmiermittel geschmiert werden, dass über einen Vorlauf Schmiermittel aus einem Vorratsbehälter entnommen und der oder jeder zu schmierenden Baugruppe zugeführt wird, und dass über einen Nachlauf das Schmiermittel von der jeweiligen zu schmierenden Baugruppe abgeführt wird. Erfindungsgemäß werden im Bereich des Nachlaufs der jeweiligen zu schmierenden Baugruppe mit Hilfe eines Sensors metallische Partikel im Schmiermittel detektiert, wobei auf Grundlage dieser Messung auf den Zustand der jeweiligen zu schmierenden Baugruppe geschlossen und gegebenenfalls eine Reparatur oder ein Austausch der jeweiligen zu schmierenden Baugruppe initiiert wird.

## Beschreibung

Die Erfindung betrifft ein Verfahren zum Betreiben einer Druckmaschine nach dem Oberbegriff des Anspruchs 1.

Druckmaschinen, wie z. B. Rollendruckmaschinen und Bogendruckmaschinen, verfügen über eine Vielzahl von Baugruppen, die während des Betriebs der Druckmaschine mit Schmiermittel geschmiert werden müssen. Bei diesen während des Betriebs zu schmierenden Baugruppen einer Druckmaschine handelt es sich z. B. um Getriebe und Lager, wie z. B. Wälzlager. Während des Betriebes einer Druckmaschine unterliegen die geschmierten Baugruppen derselben einem Verschleiß. Dann, wenn ein Verschleiß der geschmierten Baugruppen nicht rechtzeitig erkannt wird, kann es zum Ausfall der Baugruppen während des Auflagendrucks kommen, wobei dann die Gefahr besteht, dass weitere Baugruppen der Druckmaschine in Mitleidenschaft gezogen werden. Hierdurch wird die Betriebssicherheit der Druckmaschine verringert.

Bislang sind keine Verfahren bekannt, mit welchen der Zustand von geschmierten Baugruppen einer Druckmaschine überwacht bzw. detektiert werden kann. Demnach wird in festen Betriebsstundenrastern eine Wartung bzw. ein Austausch geschmierter Baugruppen an Druckmaschinen durchgeführt. Hierbei kann es vorkommen, dass noch intakte Baugruppen ausgetauscht werden. Hierdurch wird die Wirtschaftlichkeit im Druckmaschinenbetrieb reduziert.

Hiervon ausgehend liegt der vorliegenden Erfindung die Aufgabe zugrunde, ein Verfahren zum Betreiben einer Druckmaschine zu schaffen, mit welchem die Betriebsicherheit und Wirtschaftlichkeit erhöht werden kann.

Diese Aufgabe wird durch ein Verfahren zum Betreiben einer Druckmaschine gemäß Anspruch 1 gelöst. Erfindungsgemäß werden im Bereich des Nachlaufs der jeweiligen zu schmierenden Baugruppe mit Hilfe eines Sensors metallische Partikel im Schmiermittel detektiert, wobei auf Grundlage dieser Messung auf den Zustand der jeweiligen zu schmierenden Baugruppe geschlossen und gegebenenfalls eine Reparatur oder ein Austausch der jeweiligen zu schmierenden Baugruppe initiiert wird.

Mit der hier vorliegenden Erfindung wird erstmals vorgeschlagen, beim Betrieb einer Druckmaschine im Bereich des Nachlaufs geschmierter Baugruppen mit Hilfe eines Sensors metallische Partikel im Schmiermittel zu detektieren und auf Grundlage dieser Messung den Zustand der jeweiligen geschmierten Baugruppe zu beurteilen. Hierdurch kann für jede geschmierte Baugruppe individuell deren Zustand überwacht werden, um sodann für jede geschmierte Baugruppe individuell eine Reparatur, Wartung oder einen Austausch derselben zu initiieren. Hierdurch ist es demnach möglich, unter Bereitstellung einer hohen Betriebssicherheit jede geschmierte Baugruppe der Druckmaschine bis zu ihrer individuellen Lebensdauer maximal auszunutzen und so die Wirtschaftlichkeit zu erhöhen.

Bevorzugte Weiterbildungen der Erfindung ergeben sich aus den Unteransprüchen und der nachfolgenden Beschreibung. Ein Ausführungsbeispiel der Erfindung wird, ohne hierauf beschränkt zu sein, an Hand der Zeichnung näher erläutert. Dabei zeigt:

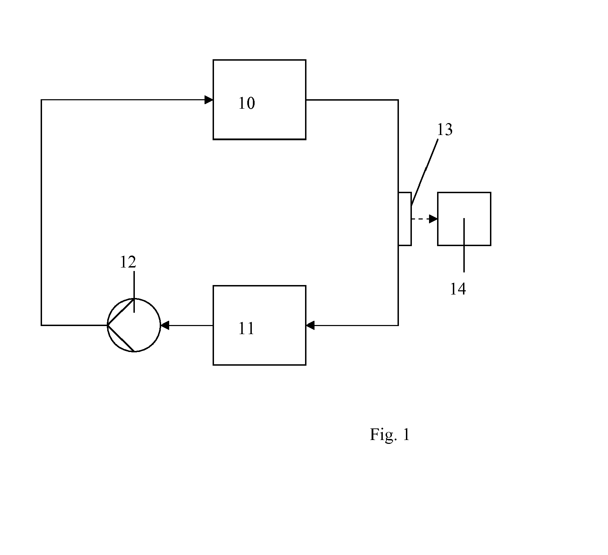

- Fig. 1:: ein Blockschaltbild zur Verdeutlichung des erfindungsgemäßen Verfahrens.

Die Erfindung betrifft ein Verfahren zum Betreiben einer Druckmaschine, wobei die Druckmaschine mindestens eine geschmierte Baugruppe umfasst. Das erfindungsgemäße Verfahren kann bei beliebigen Druckmaschinen, so z. B. bei Rollendruckmaschinen und Bogendruckmaschinen, zum Einsatz kommen.

Beim verwendeten Schmiermittel handelt es sich insbesondere um Öl, die oder jede geschmierte Baugruppe der Druckmaschine ist demnach als ölgeschmierte Baugruppe ausgeführt. Bei den ölgeschmierten Baugruppen der Druckmaschine handelt es sich um Getriebe und/oder Lager.

Die Erfindung wird nachfolgend unter Bezugnahme auf Fig. 1 beschrieben, wobei in Fig. 1 exemplarisch eine ölgeschmierte Baugruppe 10 einer Druckmaschine dargestellt ist. Im Betrieb wird der ölgeschmierten Baugruppe 10 über einen Vorlauf Schmiermittel zugeführt, wobei hierzu das Schmiermittel aus einem Vorratsbehälter 11 mit Hilfe einer Pumpe 12 entnommen und in Richtung auf die ölgeschmierte Baugruppe 10 gefördert wird. Über einen Nachlauf wird das Schmiermittel von der ölgeschmierten Baugruppe 10 abgeführt und im Ausführungsbeispiel der Fig. 1 zur Bereitstellung eines geschlossenen Schmiersystems dem Vorratsbehälter 11 zugeführt.

Im Bereich des Nachlaufs der geschmierten Baugruppe 10 ist ein Sensor 13 positioniert, wobei mit Hilfe des Sensors 13 im Bereich des Nachlaufs der ölgeschmierten Baugruppe 10 metallische Partikel im Schmiermittel detektiert werden. Der Sensor 13 übermittelt sein Messergebnis an eine Auswerteeinrichtung 14, in welcher auf Grundlage des Messsignals des Sensors 13 auf den Zustand der ölgeschmierten Baugruppe 10 geschlossen und gegebenenfalls eine Reparatur bzw. Wartung oder gar ein Austausch derselben initiiert wird. Hierdurch ist es möglich, für die ölgeschmierte Baugruppe 10 individuelle Wartungsintervalle zu initiieren und dieselbe bis zu ihrer maximalen Lebensdauer zu betreiben. Ein Verschleiß der ölgeschmierten Baugruppe 10 kann so rechtzeitig erkannt werden, um einen Ausfall derselben im Auflagendruck zu vermeiden.

Als Sensor 13 wird vorzugsweise ein induktiver Sensor verwendet, der auf einen induktiven Messprinzip beruht.

Die Auswerteeinrichtung 14 zählt auf Basis des vom Sensor 13 bereitgestellten Messsignals die metallischen Partikel im Schmiermittel des Nachlaufs, wobei auf Grundlage der so ermittelten Anzahl der metallischen Partikel im Schmiermittel auf den Zustand der ölgeschmierten Baugruppe 10 geschlossen wird.

Fig. 1 verdeutlicht die Erfindung exemplarisch am Beispiel einer einzigen ölgeschmierten Baugruppe 10. Typischerweise sind jedoch mehrere ölgeschmierte Baugruppen vorhanden, die entweder aus einem gemeinsamen Vorratsbehälter 11 oder aus individuellen Vorratsbehältern 11 mit Schmiermittel versorgt werden können. Vorzugsweise ist jeder ölgeschmierten Baugruppe ein individueller Sensor 13 zugeordnet, um für jede Baugruppe individuell im Bereich des Nachlaufs die im Schmiermittel vorhandenen metallischen Partikel zu detektieren.

Mit der hier vorliegenden Erfindung wird demnach eine zustandsorientierte Instandhaltung von ölgeschmierten Baugruppen einer Druckmaschine vorgeschlagen. Erfindungsgemäß wird der Zustand von ölgeschmierten Baugruppen über den Metallpartikelabrieb beurteilt, wobei der Metallpartikelabtrieb über geeignete Sensoren im Nachlauf des Schmiermittels gezählt und damit erfasst wird. Über die Anzahl der so gezählten Metallpartikel wird der Zustand der ölgeschmierten Baugruppe beurteilt und eine Reparatur bzw. Wartung oder gar ein Austausch derselben initiiert.

Es wird ausdrücklich darauf hingewiesen, dass der (Partikel-)sensor in einer Bypassanordnung bzw. Nebenleitung zum eigentlichen Rücklauf angeordnet sein kann. Es wird dann nur ein Teil der zurücklaufenden Schmiermittelmenge überwacht. Aus der mit dem Sensor ermittelten Partikelkonzentration kann aber trotzdem ein Rückschluss auf den Zustand der zu schmierenden Baugruppe getroffen werden.

### Bezugszeichenliste

- 10: Baugruppe
- 11: Vorratsbehälter
- 12: Pumpe
- 13: Sensor
- 14: Auswerteeinrichtung

## Patentansprüche

1. Verfahren zum Betreiben einer Druckmaschine, wobei im Betrieb der Druckmaschine geschmierte Baugruppen derselben derart mit Schmiermittel geschmiert werden, dass über einen Vorlauf Schmiermittel aus einem Vorratsbehälter entnommen und der oder jeder zu schmierenden Baugruppe zugeführt wird, und dass über einen Nachlauf das Schmiermittel von der jeweiligen zu schmierenden Baugruppe abgeführt wird, **dadurch gekennzeichnet, dass** im Bereich des Nachlaufs der jeweiligen zu schmierenden Baugruppe mit Hilfe eines Sensors metallische Partikel im Schmiermittel detektiert werden, und dass auf Grundlage dieser Messung auf den Zustand der jeweiligen zu schmierenden Baugruppe geschlossen und gegebenenfalls eine Reparatur oder ein Austausch der jeweiligen zu schmierenden Baugruppe initiiert wird.

2. Verfahren nach Anspruch 1, **dadurch gekennzeichnet, dass** als Schmiermittel Öl verwendet wird.

3. Verfahren nach Anspruch 1 oder 2, **dadurch gekennzeichnet, dass** als zu schmierenden Baugruppe Getriebe und/oder Lager ölgeschmiert werden.

4. Verfahren nach einem der Ansprüche 1 bis 3, **dadurch gekennzeichnet, dass** als Sensors ein induktiver Sensor verwendet wird, auf Basis dessen Messsignal im Bereich des Nachlaufs metallische Partikel im Schmiermittel gezählt werden, wobei auf Grundlage der Anzahl der metallischen Partikel im Schmiermittel auf den Zustand der jeweiligen zu schmierenden Baugruppe geschlossen wird.
